# FASCICULE DE BREVET EUROPEEN

(11) **EP 1 732 567 B1**
(45) Date de publication et mention de la délivrance du brevet: **08.10.2008**
(21) Numéro de dépôt: 05744629.6
(22) Date de dépôt: 25.03.2005
(51) Int. Cl.: A61K 31/5415, A61K 45/06, A61P 27/16

(54) **UTILISATION D'UN DERIVE DE LA PHENOTHIAZINE POUR LA PREVENTION ET/OU LE TRAITEMENT DE LA PERTE D'AUDITION**
VERWENDUNG EINES PHENOTHIAZIN-DERIVATS ZUR PRÄVENTION UND/ODER BEHANDLUNG VON GERHÖRVERLUST
USE OF A PHENOTHIAZINE DERIVATIVE FOR PREVENTING AND/OR TREATING HEARING LOSS

(30) Priorité: 29.03.2004 FR 0403203; 14.06.2004 FR 0406404
(43) Date de publication de la demande: 20.12.2006
(73) Titulaire: Societe de Conseils de Recherches et d'Applications Scientifiques (S.C.R.A.S) SAS, 75016 Paris (FR)
(72) Inventeur: PIGNOL, Bernadette, F- 75012 Paris (FR); PUEL, Jean-Luc, F-34660 Cournonterral (FR); AUVIN, Serge, F-91120 Palaiseau (FR); CHABRIER de LASSAUNIERE, Pierre-Etienne, F-75016 Paris (FR); WANG, Jing, F-34400 Lunel (FR)
(74) Mandataire: Bourgouin, André
(86) Numéro de dépôt international: PCT/FR2005/000713
(87) Numéro de publication internationale: WO 2005/092345

(56) Documents cités:
- WO-A-01/32654
- WO-A-02/40016
- WO-A-20/05056551
- TAKUMIDA MASAYA ET AL: "Neuroprotection of vestibular sensory cells from gentamicin ototoxicity obtained using nitric oxide synthase inhibitors, reactive oxygen species scavengers, brain-derived neurotrophic factors and calpain inhibitors." ACTA OTO-LARYNGOLOGICA. JAN 2003, vol. 123, no. 1, janvier 2003 (2003-01), pages 8-13, XP008038195 ISSN: 0001-6489
- WANG JIAN ET AL: "Leupeptin protects sensory hair cells from acoustic trauma" NEUROREPORT, vol. 10, no. 4, 17 mars 1999 (1999-03-17), pages 811-816, XP008038205 ISSN: 0959-4965
- FRANZE ANNAMARIA ET AL: "Effect over time of allopurinol on noise-induced hearing loss in guinea pigs." INTERNATIONAL JOURNAL OF AUDIOLOGY, vol. 42, no. 4, juin 2003 (2003-06), pages 227-234, XP008038194 ISSN: 1499-2027
- SEIDMAN M D: "Effects of dietary restriction and antioxidants on presbyacusis" LARYNGOSCOPE 2000 UNITED STATES, vol. 110, no. 5 I, 2000, pages 727-738, XP008038200 ISSN: 0023-852X
- PIGNOL B (REPRINT) ET AL: "Doxorubicin (Doxo) induced muscle wasting in mice. Protective effect of BN82270 , an hybrid molecule with dual activity (calpain inhibitor and antioxidant) on cachexia" NEUROMUSCULAR DISORDERS, (SEP 2004) VOL. 14, NO. 8-9, PP. 611-611. ISSN: 0960-8966. PB - PERGAMON-ELSEVIER SCIENCE LTD, THE BOULEVARD, LANGFORD LANE, KIDLINGTON, OXFORD OX5 1GB, ENGLAND., septembre 2004 (2004-09), XP002337502
- AUVIN SERGE ET AL: "Novel dual inhibitors of calpain and lipid peroxidation" BIOORGANIC & MEDICINAL CHEMISTRY LETTERS, vol. 14, no. 14, 16 juillet 2004 (2004-07-16), pages 3825-3828, XP002304686 ISSN: 0960-894X
- YAMASHITA D ET AL: "Delayed production of free radicals following noise exposure" BRAIN RESEARCH 03 SEP 2004 NETHERLANDS, vol. 1019, no. 1-2, 3 septembre 2004 (2004-09-03), pages 201-209, XP008038192 ISSN: 0006-8993

## Description

La présente invention concerne l'utilisation d'un dérivé de la phénothiazine présentant une activité inhibitrice des calpaïnes et une activité piégeuse des formes réactives de l'oxygène (ROS pour "reactive oxygen species") pour la préparation d'un médicament destiné à prévenir et/ou traiter la perte d'audition. Un produit comprenant au moins un tel composé et une composition pharmaceutique le contenant sont également divulgués.

Parmi les nombreuses causes de la perte d'audition, on peut notamment citer les maladies telles que méningite ou otite, les causes d'ordre génétique, les blessures, les tumeurs, les drogues, l'administration de médicaments tels que certains antibiotiques, anticancéreux, agents anti-inflammatoires non stéroïdiens, diurétiques, ulcéreux ou anticonvulsivants, l'exposition prolongée aux solvants organiques aromatiques tels que toluène ou xylène, le vieillissement et l'exposition au bruit. La presbyacousie (surdité liée au vieillissement), l'exposition prolongée au bruit et l'administration de médicaments sont les principales causes de la perte d'audition.

Il est aujourd'hui reconnu que certains antibiotiques de la famille des aminoglycosides, comme la gentamycine et la tobramycine, qui sont utilisés dans le traitement de graves infections sont responsables de la surdité cochléaire. La toxicité aux aminosides (aminoglycosides tels que l'amikacine, la dibékacine, la gentamicine, l'isépamicine, la nétilmicine, la spectinomycine, la tobramycine) se manifeste tout d'abord par une atteinte de l'audition sur les hautes fréquences et n'est pas reconnue d'emblée par le patient. Ce n'est que progressivement que celui-ci se trouvera gêné. Elle est malheureusement souvent irréversible.

Le bruit qui nous entoure abaisse notre audition. La perte d'audition due au bruit se produit lorsque les cellules ciliées qui transmettent le son vers l'oreille interne sont endommagées et ne peuvent plus ordonner au nerf auditif d'envoyer des impulsions électriques vers le cerveau.

L'intensité du son et la durée d'exposition sont les deux principaux facteurs influençant la perte d'audition. Bien que la réaction à l'exposition au bruit varie d'une personne à l'autre, quelques faits peuvent être rapportés avec certitude. Des recherches ont démontré que l'exposition prolongée à 85 décibels (dB) ou plus provoquera, au fil du temps, une perte d'audition permanente.

Les statistiques Européennes et Nord-Américaines montrent que 8 à 10 % de la population est atteinte de pathologies de la cochlée (surdités, acouphènes). Compte tenu des niveaux sonores émis dans les discothèques, concerts techno et autres baladeurs, toute une génération de sourds ou d'acouphéniques est en train de se constituer. Ainsi les problèmes liés à l'âge (presbyacousie) qui débutent aujourd'hui vers 60 ans, pourraient débuter bien plus tôt, c'est-à-dire vers 35-40 ans.

Le problème des pathologies auditives est qu'elles résultent en grande majorité de la perte des cellules sensorielles et nerveuses de l'oreille interne (ou cochlée). Et ces cellules, au-delà d'une phase de développement pour l'essentiel intra-utérine, n'ont pas la capacité de se renouveler après leur dernier stade de différenciation.

La perte progressive du capital sensitif et nerveux de l'oreille liée à différentes pathologies cochléaires semble, encore aujourd'hui, hors de portée de tout traitement.

La demande WO 02/40016 décrit l'utilisation d'une association d'inhibiteurs de calpaïne et de piégeurs des formes réactives de l'oxygène pour le traitement de pathologies où ces enzymes et/ou ces espèces radicalaires sont impliquées, comme la perte d'audition.

L'invention a donc pour objet l'utilisation d'un dérivé hétérocyclique répondant à la formule (I) 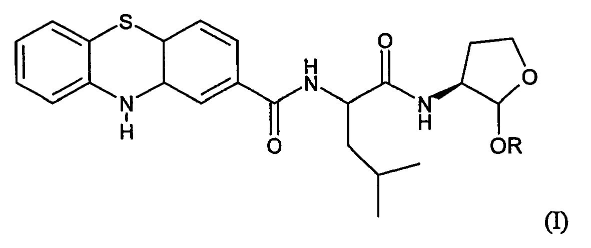 sous forme de diastéréoisomères ou toutes combinaisons de ces formes,
dans laquelle R représente l'atome d'hydrogène, un radical (C₁-C₆)alkyle, arylalkyle ou -C(O)R' dans lequel R' représente un radical hétérocycloalkyle, (C₁-C₆)alkyle, aryle ou aralkyle ;
les radicaux alkyle, aryle ou hétérocycloalkyle étant éventuellement substitués par un ou plusieurs substituants identiques ou différents choisis parmi : (C₁-C₆)alkyle, hydroxy, (C₁-C₆)alkoxy, nitro, cyano, halogène ou -NR₁R₂ ;
R₁ et R₂ représentent, indépendamment, un atome d'hydrogène ou un radical (C₁-C₆)alkyle, ou bien R₁ et R₂ forment ensemble avec l'atome d'azote auquel ils sont rattachés un hétérocycle éventuellement substitué,
pour la préparation d'un médicament destiné à prévenir et/ou traiter la perte d'audition.

Dans la présente demande, il faut comprendre par "traumatisme" un ensemble de lésions locales intéressant les tissus et les organes, provoqué par un agent extérieur. Dans le cas d'un traumatisme acoustique, l'agent extérieur est principalement le bruit.

Par (C₁-C₆)alkyle, on entend un radical alkyle linéaire ou ramifié comptant de 1 à 6 atomes de carbone comme, par exemple, les radicaux méthyle, éthyle, propyle, isopropyle, butyle, isobutyle, sec-butyle et tert-butyle, pentyle, néopentyle, isopentyle, hexyle, isohexyle. Les radicaux (C₁-C₆)alkoxy peuvent correspondre aux radicaux alkyle indiqués ci-dessus comme par exemple les radicaux méthoxy, éthoxy, propyloxy ou isopropyloxy mais également butoxy linéaire, secondaire ou tertiaire. Les radicaux alkylcarbonyle peuvent correspondre aux radicaux alkyle indiqués ci-dessus comme par exemple les radicaux méthylcarbonyl, éthylcarbonyl, propylcarbonyl. Par halogène, on entend les atomes de fluor, de chlore, de brome ou d'iode.

Par aryle, on entend un système carbocyclique ou hétérocyclique comprenant au moins un cycle aromatique, un système étant dit hétérocyclique lorsque l'un au moins des cycles qui le composent comporte un hétéroatome (O, N ou S). Comme exemple de radical aryle carbocyclique, on peut citer phényle ou naphtyle. Comme exemple de radical aryle hétérocyclique (ou hétéroaryle), on peut citer thiényle, furyle, pyrrolyle, imidazolyle, pyrazolyle, isothiazolyle, thiazolyle, isoxazolyle, oxazolyle, pyridyle, pyrazinyle, pyrimidyle, benzothiényle, benzofuryle et indolyle.

Le terme hétérocycle (ou hétérocycloalkyle), représente de préférence un hétérocycle mono ou bicyclique, saturé ou insaturé, comportant de 1 à 5 hétéroatomes choisis parmi O, S et N. L'atome d'azote peut éventuellement être substitué par un radical choisi parmi : alkyle, aryle, aralkyle et alkylcarbonyle. Comme exemple d'hétérocycle saturé, on peut citer : tétrahydrofuranne, tétrahydropyranne, oxétane, oxépane, tétrahydrothiophène, tétrahydrothiopyranne, thiétane, pyrrolidine, pipéridine, azétidine, 1,3-dioxanne, 1,3-dioxolanne, 1,3-dithiolanne, 1,3-dithianne, 1,3-oxathiolanne, 1,3-oxazolidine, 1,3-imidazolidine ou 1,3-thiazolidine. Comme exemple d'hétérocycle insaturé, on peut citer : dihydrothiophène, dihydrofurane, dihydropyrrole, dihydroimidazole, dihydropyrazole, dihydropyridine, indoline.

Les radicaux arylalkyles (ou aralkyles) désignent les radicaux dans lesquels respectivement les radicaux aryle et alkyle sont tels que définis ci-dessus comme par exemple benzyle, phenéthyle ou naphtylméthyle.

Dans le cas du radical de formule -NR₁R₂ où R₁ et R₂ forment ensemble avec l'atome d'azote auquel ils sont rattachés un hétérocycle éventuellement substitué, l'hétérocycle est de préférence saturé et comprend de 4 à 7 chaînons et de 1 à 3 hétéroatomes incluant l'atome d'azote déjà présent, les hétéroatomes supplémentaires étant choisis indépendamment dans le groupe constitué des atomes O, N et S. Ledit hétérocycle peut être, par exemple, le cycle azétidine, pyrrolidine, pipéridine, pipérazine, morpholine ou thiomorpholine. Ledit hétérocycle peut être substitué par un ou plusieurs substituants identiques ou différents choisis parmi le groupe hydroxy, un radical alkyle, aryle, aralkyle ou alkoxy ou un atome d'halogène.

L'invention a plus particulièrement pour objet une utilisation telle que définie ci-dessus, caractérisée en ce que R représente -C(O)R' et de préférence R' représente un radical alkyle.

De manière très préférentielle, le composé (I) est caractérisé en ce que R représente -C(O)-CH₃. Ce dernier composé sera appelé par la suite composé (1).

L'invention a plus particulièrement pour objet également une utilisation telle que définie ci-dessus, caractérisée en ce que R représente l'hydrogène.

De manière très préférentielle également, le composé (I) tel que défini ci-dessus a pour formule 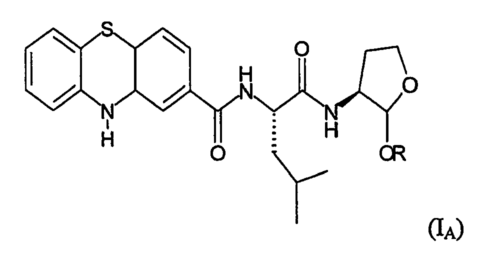 et plus particulièrement l'une des formules suivantes

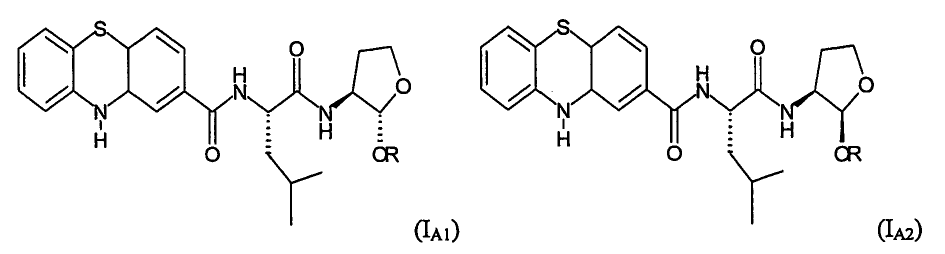

De manière très préférentielle également, le composé (I) tel que défini ci-dessus a pour formule 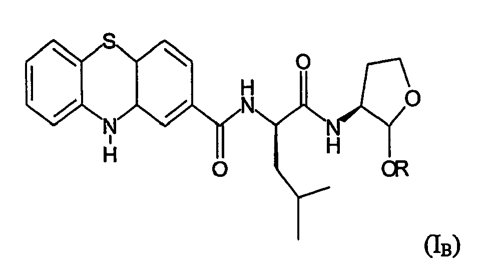 et plus particulièrement l'une des formules suivantes

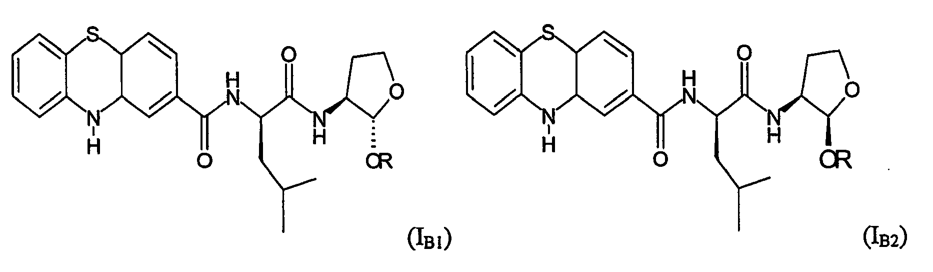

Les composés tel que définis ci-dessus, sont de nouveaux agents protecteurs combinant des effets antioxydants et anti-calpaïnes et décrits dans la demande WO 01/32654.

La présente invention a donc pour objet également une utilisation d'un composé de formule (I) telle que définie ci-dessus, en pré- ou post-traitement par rapport à l'origine de la perte d'audition.

La présente invention a pour objet également une utilisation d'un composé de formule (I) telle que définie ci-dessus, pour la préparation d'un médicament destiné à prévenir et/ou traiter la perte d'audition suite à l'administration d'un autre médicament. De préférence, l'autre médicament est un antibiotique tel que la gentamicine, un anticancéreux tel que le cisplatine, un agent anti-inflammatoire non stéroïdien tel que les dérivés de l'acide salicylique ou l'ibuprofène, un diurétique tel que la furosémide, un anti-ulcéreux tel que la cimétidine ou l'oméprazole, un agent anticonvulsivant tel que la carbamazépine ou l'acide valproique. De manière très préférentielle, l'autre médicament est un antibiotique et plus particulièrement la gentamicine.

La présente invention a pour objet également une utilisation d'un composé de formule (I) telle que définie ci-dessus, pour la préparation d'un médicament destiné à prévenir et/ou traiter la perte d'audition suite à la presbyacousie.

La présente invention a pour objet également une utilisation d'un composé de formule (I) telle que définie ci-dessus, pour la préparation d'un médicament destiné à prévenir et/ou traiter la perte d'audition suite à un traumatisme acoustique.

Les composés selon l'invention peuvent être utilisés seuls ou en association avec au moins une autre substance susceptible de prévenir et/ou traiter la perte d'audition ou bien de prévenir et/ou traiter toutes pathologies associées à la perte d'audition, choisie parmi des anti-oxydants, des inhibiteurs de calpaïnes tels que la leupeptine ou Neurodur, des vasodilatateurs périphériques tel que l'EGb 761^{®}, des agonistes ou antagonistes du récepteur NMDA, des inhibiteurs peptidiques de c-Jun N-Terminal Kinase tel que D-JNK-1.

L'invention a également pour objet l'utilisation telle que définie ci-dessus, caractérisée en ce qu'il est associé à au moins une autre substance à activité pharmaceutique choisie parmi : des anti-oxydants, des inhibiteurs de calpaïnes, des vasodilatateurs périphériques, des agonistes ou antagonistes du récepteur NMDA, des inhibiteurs peptidiques de c-Jun N-Terminal Kinase.

Un composé de formule (I) et de préférence le composé (1) tel que défini ci-dessus, peut être administré à une dose comprise entre 50 à 500 µM en application locale. Dans le cas d'un traitement local intracochléaire, il peut être administré à une dose comprise entre 50 et 200 µM. Dans le cas d'un traitement local extracochléaire, il peut être administré à une dose comprise entre 200 et 500 µM. Les substances qui leur sont éventuellement associées, connues en pharmacologie, sont administrées aux doses habituellement conseillées.

Les composés tels que définis ci-dessus ainsi que les substances à activité pharmaceutique qui leur sont éventuellement associées, peuvent être administrés par les voies classiques d'administration telle que orale, intrapéritonéale, sous-cutanée ou intraveineuse. Elles peuvent être administrées simultanément ou séparément, par des voies d'administration identiques ou différentes. De préférence, le composé (I) tel que défini ci-dessus est administré localement par les techniques communément utilisées dans le traitement de l'oreille interne telles que des microcathéters, des seringues pour injection transtympanique ou des tubes équipés de mèche de type Silverstein Microwick^{™}.

Un produit comprenant le dérivé hétérocyclique de formule (I) telle que définie ci-dessus, sous forme de diastéréoisomères ou toutes combinaisons de ces formes, et au moins une substance à activité thérapeutique, en tant que produit de combinaison pour une utilisation simultanée, séparée ou étalée dans le temps pour prévenir et/ou traiter la perte d'audition est également décrit. De préférence, il s'agit d'un produit tel que défini ci-dessus, pour prévenir et/ou traiter la perte d'audition, soit suite à l'administration d'un médicament, et plus particulièrement un antibiotique, soit suite à la presbyacousie, soit suite à un traumatisme acoustique.

Enfin, à titre de médicament, un produit tel que défini ci-dessus est divulgué. Dans le cas de la perte d'audition suite à l'administration d'un médicament, ce dernier est de préférence un antibiotique, et de manière préférentielle la gentamicine.

Les substances à activité pharmaceutique qui lui sont éventuellement associées sont administrées par les voies d'administration habituellement envisagées pour ces substances dans le domaine thérapeutique considéré.

Dans le cas de la perte d'audition due à un traumatisme acoustique, l'administration d'un composé (I) tel que défini ci-dessus, peut être effectuée quelques jours avant le traumatisme acoustique de préférence 2 à 3 jours avant le traumatisme, et 24 heures après le traumatisme. De préférence, cette administration est effectuée dans les 7 premières heures suivant le traumatisme. De préférence également, cette administration peut être effectuée dans les 2 heures qui suivent le traumatisme.

L'invention a donc pour objet l'utilisation décrite ci-dessus, caractérisée en ce que le composé (1), sous forme de diastéréoisomères ou toutes combinaisons de ces formes, est administré dans les 7 heures et de préférence dans l'heure suivant le traumatisme.

Les résultats montrant l'efficacité thérapeutique du composé (1) sur la récupération fonctionnelle après traumatisme acoustique sont présentés dans la partie expérimentale.

Les exemples suivants sont présentés pour illustrer les procédures ci-dessus.

### PARTIE EXPÉRIMENTALE :

### Etude pharmacologique

### 1) Ototoxicité induite après un traitement à la gentamicine

### ◆ Démonstration de l'effet protecteur du composé (1), administré en co-traitement, vis-à-vis de la perte des cellules ciliées induite par la gentamicine.

Il est prouvé que la gentamicine et autres aminoglycosides provoquent un dommage des cellules ciliées et une perte d'audition chez l'être humain. Les poissons Zebra montrent les organes sensoriels à la surface de leur corps, appelés neuromasts. Chez ces poissons, les cellules ciliées neuromasts peuvent être marquées avec du DASPEI et ce marquage reflète le nombre de cellules ciliées. Ces cellules ciliées sont structurellement et fonctionnellement similaires aux cellules ciliées internes de l'oreille humaine.

Le dommage des cellules ciliées internes est induite chez des poissons Zebra par la gentamicine. Pour tester l'effet du composé (1) sur la protection des cellules ciliées endommagées par la gentamicine, le composé (1) a été administré en co-traitement avec la gentamicine. Les cellules ciliées internes sont ensuite marquées et quantifiées.

L'étude est faite sur des poissons âgés de 5 jours incubés avec 1 µg/ml de gentamicine pendant 24 heures en présence ou en absence du composé (1). Des contrôles sont effectués en parallèle ; véhicule seul (1 % DMSO ; contrôle positif). Les poissons traités par la gentamicine sont les contrôles négatifs.

La coloration DASPEI (2,4-dimethyl-aminostyryl-N-ethyl pyridinium iodide) est effectuée pour visualiser les cellules ciliées *in vivo* (n=5 par groupe). L'analyse morphométrique est utilisée pour quantifier le signal de marquage des cellules ciliées. Le signal de coloration DASPEI des contrôles positifs a été défini comme le 100 %.

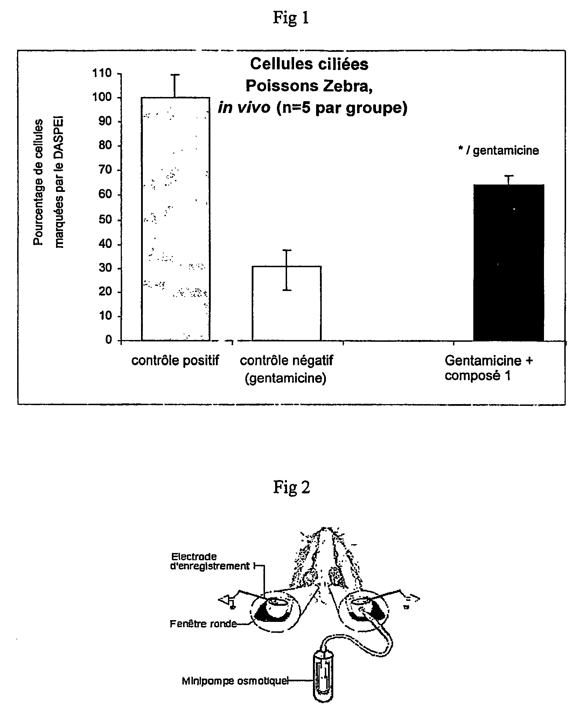

Les résultats sont présentés dans la Figure 1 (Pourcentage de cellules ciliées marquées par le DASPEI : contrôle positif (Zebra - 1 % DMSO) ; contrôle négatif (Zebra - 1 % DMSO - gentamicine 1 µg/ml) et effet du produit (Zebra - 1 % DMSO - gentamicine - composé (1)). Expérience effectuée sur 5 animaux par groupe).

Ces résultats montrent que :
- le signal de coloration du contrôle négatif représente 31,5 ± 4,2 % du signal contrôle, soit une perte de 68,5 ± 4,9 % des cellules ciliées suite au traitement par la gentamicine ;
- le signal de coloration des animaux traités par la gentamicine et le composé (1) représente 65,2 ± 4,4 % du signal contrôle soit une protection hautement significative de 48,7 ± 2,63 % des cellules ciliées endommagées par le traitement à la gentamicine.

### 2) Perte d'audition après un trauma acoustique

Il s'agit d'étudier chez le cochon d'inde, les effets protecteurs en pré- et post- traitement du composé (1), sur la protection des cellules ciliées de l'oreille interne d'une part et la récupération fonctionnelle auditive d'autre part, suite à une perte d'audition induite par un trauma acoustique. Le composé (1) a été administré par voie locale "intra et extra cochléaire" de manière à se rapprocher de son utilisation en clinique chirurgicale humaine.

La récupération fonctionnelle obtenue grâce à ce nouvel agent protecteur est quantifié à l'aide d'un test fonctionnel qui est la mesure de l'audiogramme des animaux. Cet audiogramme est réalisé grâce à l'enregistrement de l'activité du potentiel action composite du nerf auditif. Les audiogrammes sont enregistrés avant et après le traumatisme acoustique. Des analyses en microscopie électronique à balayage complètent ces données fonctionnelles par une étude des pertes cellulaires le long de la spirale cochléaire ainsi que l'effet protecteur du composé (1).

Toutes les expériences sont réalisées sur des cochons d'inde ; chaque expérience est réalisée selon un schéma similaire.

Le schéma général d'une phase expérimentale est le suivant :
- Les animaux sont anesthésiés grâce à une injection intramusculaire d'un mélange de Rompun^{®} à 2 % (3 mg/kg) + Zolutil^{®} (40 mg/kg). Cette anesthésie présente l'avantage de se dissiper rapidement et peut être maintenue plusieurs heures grâce à l'injection régulière (toutes les 2 heures) du tiers de la dose initiale.
- La mise en place des électrodes et de la mini pompe selon la Figure 2.

L'approche de la cochlée est réalisée par un abord dorsal. Après avoir rasé et nettoyé le scalp, une incision de 2 cm est effectuée derrière le pavillon de l'oreille. La parotide et les plans musculaires recouvrant la bulle tympanique sont réclinés. Une fois séché et nettoyé, l'os est percé au niveau de sa paroi supérieure sous l'émergence du nerf facial. L'électrode active (fil de platine de 0,13 mm de diamètre, gainé de Téflon) est ensuite introduite dans la bulle et mise au contact de la membrane de la fenêtre ronde sous contrôle d'un microscope opératoire (WILD M650). Après enregistrement des seuils audiométriques, un petit trou de 0,2 mm de diamètre est fraisé manuellement dans le tour basal de la rampe tympanique juste au dessous la fenêtre ronde.

Une pipette de verre (0,1 mm de diamètre à la pointe), connectée à la micro pompe par un cathéter, est insérée dans la cochlée à l'aide d'un deuxième micromanipulateur. La bulle tympanique, incluant l'électrode d'enregistrement et la pipette de perfusion, est refermée avec de la résine dentaire.

La micro pompe est glissée sous la peau de l'animal, les plans musculaires et cutanés sont badigeonnés de Bétadine^{®}, suturés avec du fil résorbable et recouverts avec une solution antibiotique (Rifocine 5P100^{®}).

Une seconde incision d'environ 1,5 cm est ensuite effectuée sur le vertex en vue de fixer un connecteur (connectral, ref. : 8/45-05.050.000) sur le crâne de l'animal. Après avoir gratté le périoste de l'os, cette zone est soigneusement séchée, badigeonnée de nitrate d'argent puis recouverte d'un film de cyanolite.

Les électrodes (active et de référence), sont glissées sous la peau jusqu'au connecteur sur lequel elles sont soudées. Le connecteur est alors fixé sur le crâne avec de la résine dentaire.

### Techniques de stimulations :

Les stimulations sonores seront produites grâce à deux synthétiseurs Hewlett-Packard (HP 3314 A et HP 8904 A) et délivrées en champ libre par l'intermédiaire d'un haut parleur (JBL 075) placé à 10 cm de l'oreille. La calibration du système acoustique est réalisée dans une oreille artificielle en utilisant un microphone de 1/2 pouce (type 4134, Bruel et Kjaer) et un amplificateur de mesure (type 2606) permettant de lire directement le niveau sonore en décibel SPL (dB SPL, référence : 2,10⁻⁵ Pa). Afin de visualiser les signaux acoustiques, la sortie de l'amplificateur de mesure est connectée à un oscilloscope. Les animaux sont exposés à un son de 6 kHz, à 120 dB SPL durant 30 minutes.

### Techniques d'enregistrement :

Les potentiels cochléaires enregistrés à partir de l'électrode implantée dans la cochlée *via* le connecteur fixé sur la tête de l'animal sont amplifiés (gain 1000) et filtrés (32 Hz - 3200 Hz) à l'aide d'un préamplificateur et d'un amplificateur différentiel de type GRASS P 511 K. La trace directe est visualisée sur un oscilloscope (Tektronix type 513). Ce signal est moyenné (256 passages) pour réduire le bruit de fond, et stocké sur un ordinateur PC 486, 66 mégahertz (Hewlett-Packard-Vectra 05/65). Le critère de seuil est défini comme étant la valeur en dB SPL nécessaire pour évoquer une réponse mesurable (> 2 µV). Deux électrodes placées au contact de la cochlée (fenêtre ronde) permettent d'enregistrer les potentiels cochléaires et de réaliser les audiogrammes pour chaque oreille. Les seuils audiométriques sont enregistrés 20 minutes après le traumatisme sonore et quotidiennement durant un mois.

### Implication de l'activation de la calpaïne après un trauma acoustique :

Le clivage d'un substrat spécifique de la calpaïne, la fodrine, est quantifié pour déterminer l'activation de l'enzyme calpaïne après un trauma acoustique. La calpaïne clive la fodrine de 240 KD pour former un produit de dégradation de 150 KD. Un double marquage est réalisé avec un anticorps polyclonal spécifique du fragment 150 KD et un anticorps anti calbindin permettant d'identifier les cellules ciliées. La fluorescence est visualisée par un microscope à confocal.

### Mécanismes moléculaires de la mort des cellules ciliées après un trauma acoustique :

Pour déterminer la nature de la mort cellulaire, la fragmentation du DNA des cellules de la cochlée est quantifiée par la méthode TUNEL.

### Détermination de l'intégrité des cellules ciliées après un trauma acoustique :

L'intégrité des cellules de la cochlée est déterminée par immunocytochimie en utilisant un anticorps anti-cytochrome C. Dans les cellules saines, le cytochrome C est localisé dans les mitochondries. Après un trauma acoustique, le cytochrome C est diffus et distribué dans le cytoplasme.

### Phase 1 : Démonstration de l'effet protecteur du composé (1), administré en pré-traitement, vis à vis de la perte d'audition et la perte cellulaire le long de la spirale cochléaire induite par le traumatisme

### Protocole - Phase 1 : pré administration du composé (1)

Une mini pompe osmotique, placée sous la peau, délivre le composé (1).

Dans la cochlée via un cathéter (perfusion intra cochléaire), deux jours après l'implantation de la mini pompe, les animaux subissent le traumatisme sonore.

Cette expérience est menée sur 7 animaux puis 30 animaux pour la dose réponse.

Le composé (1) est appliqué directement dans la cochlée (perfusion intra cochléaire) *via* une mini pompe osmotique (débit de 1 µl/h, volume de 200 µl, durée de diffusion 7 jours) implantée à demeure dans la cochlée 2 jours avant le traumatisme. Cette technique permet de déterminer l'effet protecteur du composé (1), à la dose de 100 µM, vis-à-vis des pertes cellulaires le long de la spirale de la cochlée induite par le traumatisme ainsi que la récupération fonctionnelle de l'audition. Puis un effet dose du produit permet de déterminer la dose efficace permettant de préserver 50 % de l'audition.

### Résultats phase 1

Le composé (1) à la concentration de 100 µM.

### Test fonctionnel mesuré par audiogramme. Perte d'audition après un trauma acoustique et protection par le composé 1.

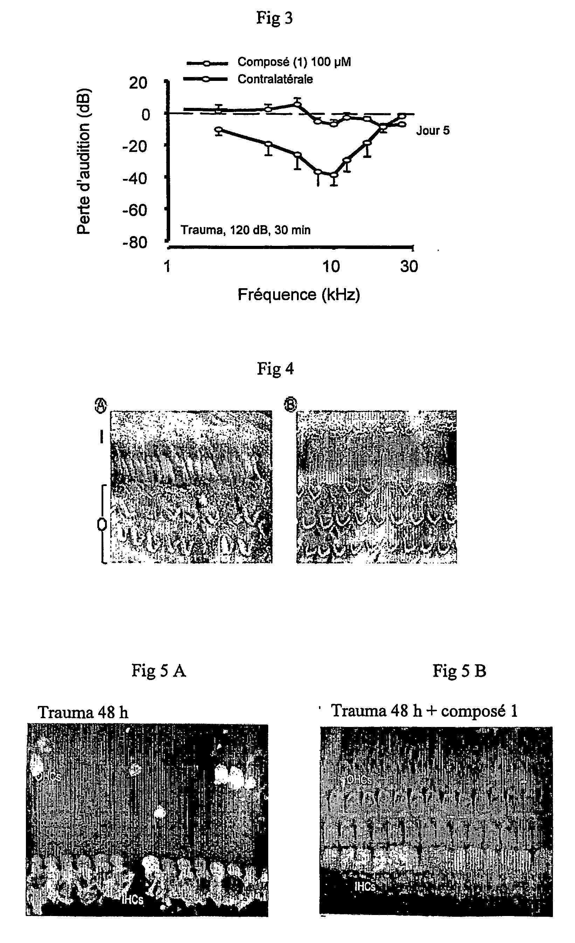

Cinq jours après un traumatisme de 120 db d'une durée de 30 min, la mesure des audiogrammes permettent de démontrer l'efficacité du composé (1) ; ce produit permet de récupérer 100 % de l'audition (Figure 3) lorsqu'il est perfusé à 100 µM, 2 jours avant ce traumatisme.

### Étude morphologique : Histologie des cellules le long de la spirale de la cochlée après un trauma acoustique. Protection par le composé (1).

A la fin de l'évaluation électrophysiologique (audiogramme), 30 jours après le trauma acoustique, les cochlées des animaux sont prélevées, et préparées pour la microscopie électronique.

Les pertes cellulaires sont déterminées grâce à un comptage des touffes ciliaires au microscope à balayage. Des données qualitatives sont recherchées en procédant à des observations forts grossissements de la surface des organes de Corti.

Après un traumatisme de 120 db d'une durée de 30 min, les cellules ciliées internes sont détruites ainsi qu'une partie des trois rangées des cellules ciliées externes. L'histologie de ces cellules permet de démontrer l'efficacité du composé (1). Ce produit permet de protéger 100 % des cellules ciliées internes et une grande partie des cellules ciliées externes lorsqu'il est perfusé à 100 µM, 2 jours avant ce traumatisme (Figure 4 : Données histologiques ; Photo A : trauma acoustique ; Photo B : composé (1) + trauma acoustique ; I = cellules ciliées internes ; O = cellules ciliées externes).

### Implication de l'activation de la calpaïne après un trauma acoustique. Inhibition par le composé 1.

L'activation de l'enzyme calpaïne est déterminée par la quantification de la dégradation d'un substrat spécifique de l'enzyme, la fodrine, en un fragment de 150 KD.

Ce clivage de la fodrine n'est jamais détecté dans les cellules de la cochlée chez des témoins.

Après un trauma acoustique, le marquage immuno du fragment 150 KD, provenant du clivage de la fodrine due à l'activation de l'enzyme calpaïne, est visible sur les cellules de la cochlée externes grâce au marquage par l'anticorps anti-FBDP ("Fodrine Breakdown products", fluorescence verte ; Figure 5A : 48 heures après le trauma acoustique). Cette activation de l'activité de la calpaïne après un trauma acoustique est associée à une perte des cellules de la cochlée visualisée par l'absence de marquage par l'anticorps anti-calbindin qui permet d'identifier les cellules ciliées intactes.

L'application locale de 100 µM du composé (1) inhibiteur de l'activation de la calpaïne et antioxydant, prévient le clivage de la fodrine par l'enzyme calpaïne dans les cellules de la cochlée exposées à un trauma acoustique. Cette absence de marquage fluorescent vert, donc de la non dégradation du substrat spécifique de la calpaïne est associé à une protection des cellules de la cochlée (marquage par anticorps anti-calbindin; Figure 5 B).

### Mécanismes moléculaires de la mort des cellules ciliées après un trauma acoustique. Protection par le composé 1.

Pour déterminer la nature de la mort des cellules de la cochlée induite après un trauma acoustique, la fragmentation du DNA (quantifiée par la méthode TUNEL) est effectuée sur des animaux ayant subi un trauma acoustique. L'effet du composé (1) est testé sur ce paramètre pour déterminer son implication sur ce mécanisme de mort cellulaire *via* l'apoptose.

Aucune cellule "TUNEL positif' n'est observée dans les cochlées obtenues à partir des animaux contrôles non exposés au bruit.

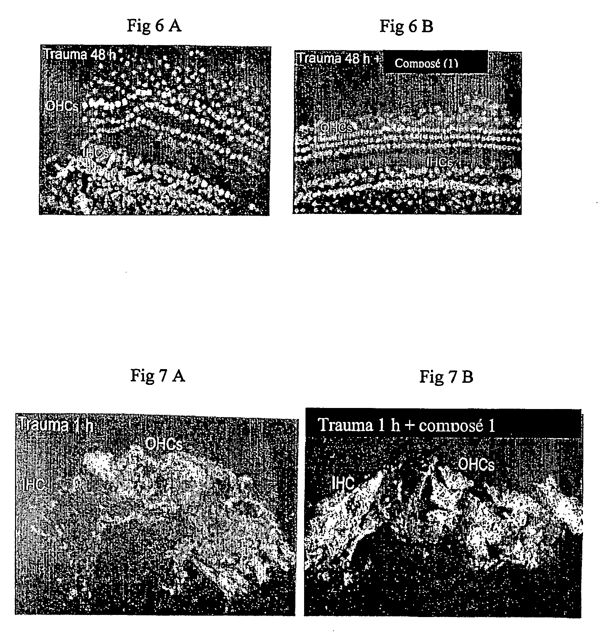

Les cochlées exposées à un trauma acoustique ont des noyaux cellulaires TUNEL positifs dans la région de l'organe de Corti (Figure 6A : 48 heures après le trauma acoustique). Les noyaux marqués sont localisés dans la région supérieure de l'organe de Corti. Il est donc vraisemblable que ces noyaux marqués appartiennent aux cellules ciliées plutôt qu'aux cellules de support. De nombreux noyaux marqués sont visibles dès une heure après le trauma acoustique et ce marquage est encore visible 4 jours après le trauma acoustique. La mort cellulaire des cellules ciliées suite à un trauma acoustique est donc obtenue *via* un mécanisme apoptotique associé à une fragmentation du DNA.

L'application locale de 100 µM du composé (1), inhibiteur de l'activation de la calpaïne et antioxydant, prévient le marquage des noyaux des cellules ciliées (méthode TUNEL, Figure 6B : composé (1) - 48 heures après le trauma acoustique). Le composé (1) supprime la mort cellulaire par apoptose induite par un trauma acoustique.

### Perte de l'intégrité des cellules ciliées après un trauma acoustique. Protection par le composé (1).

Pour déterminer la perte d'intégrité des cellules ciliées après un trauma acoustique, la diffusion du cytochrome C mesurée *via* l'utilisation d'un anticorps anti cytochrome C est effectuée sur des animaux ayant subi un trauma acoustique.

Le composé (1) est testé sur la libération du cytochrome C, du compartiment mitochondrial vers le compartiment cytoplasmique, pour déterminer son effet sur la perte d'intégrité des cellules ciliées après un trauma acoustique.

Dans les cochlées obtenues à partir des animaux contrôles non exposés au bruit, le cytochrome C est localisé dans les mitochondries.

Les cochlées exposées à un trauma acoustique montrent un marquage du cytochrome C diffus et distribué dans le cytoplasme (Figure 7 A).

L'application locale de 100 µM du composé (1), inhibiteur de l'activation de la calpaïne et antioxydant, prévient la dispersion du cytochrome C des mitochondries vers le cytoplasme des cellules ciliées exposées au trauma acoustique et donc maintient l'intégrité des cellules (Figure 7 B).

### Courbe dose-réponse du composé (1).

L'effet protecteur du composé (1) a été évalué en faisant varier la dose afin de définir une dose efficace permettant de récupérer 50 % de l'audition perdue (DE₅₀). Huit groupes de 5 animaux dont ceux recevant de la périlymphe artificielle seule, et ceux recevant 1, 3, 10, 33, 100 µM du composé (1), soit 30 animaux sont utilisés.

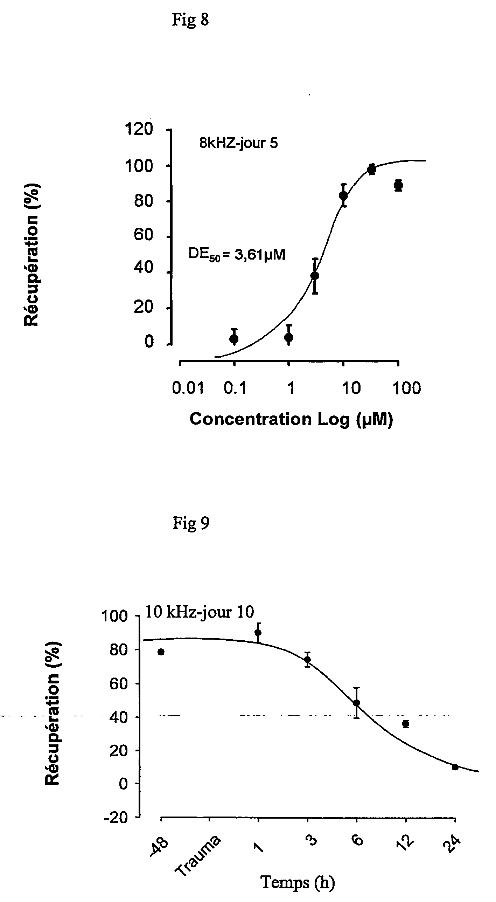

Cinq jours après un traumatisme de 120 db d'une durée de 30 min, la mesure des audiogrammes permet de déterminer la dose efficace du composé (1) (DE₅₀= 3,61 µM) permettant de récupérer 50 % de l'audition perdue après le traumatisme (Figure 8).

### Phase 2 : Démonstration de l'effet protecteur du composé (1), administré par perfusion extra cochléaire en post traitement, vis à vis de la perte cellulaire le long de la spirale cochléaire et de la perte d'audition. Détermination du temps auquel après un traumatisme, le composé (1) permet de récupérer 50 % de l'audition.

### Post administration du composé (1) :

Des études préliminaires ont montré que la cochléostomie effectuée après l'exposition aggravait les effets traumatiques du son. Ce faisant, les animaux implantés après le traumatisme récupéraient moins bien que les animaux non-implantés. Pour éliminer le traumatisme lié à la cochléostomie, nous avons mis au point une méthode non traumatique en appliquant le composé directement sur la fenêtre ronde (extracochléaire).

Une mini pompe osmotique, est implantée sous la peau et délivre le composé (1) dans la cochlée *via* un cathéter (perfusion extra cochléaire), 30 minutes ou 1, 3, 6, 12, ou 24 heures après le traumatisme sonore subi par les animaux.

Cette expérience est menée sur 30 animaux.

Le composé (1) est appliqué 48 heures avant ou 1, 3, 6, 12, ou 24 heures après le traumatisme dans l'oreille moyenne (perfusion extra cochléaire) *via* une mini pompe osmotique (débit de 1 µl/h, volume de 200 µl, durée de diffusion 7 jours). Cette mini pompe est implantée à demeure dans l'oreille moyenne et diffuse le produit directement sur la fenêtre ronde.

Ces expériences permettent de déterminer la fenêtre thérapeutique du composé (1) (FT), c'est-à-dire le temps maximum auquel le composé (1) peut être donné après un traumatisme tout en montrant un effet protecteur. Le temps efficace permettant de récupérer 50 % de l'audition perdue après le traumatisme (TE₅₀) est également déterminé. Cette technique permet également de déterminer un mois après le trauma acoustique, l'effet protecteur du composé (1), à la dose de 300 µM, vis-à-vis des pertes cellulaires le long de la spirale de la cochlée induite par le traumatisme.

### Résultats phase 2

Composé (1) à la concentration de 300 µM.

### Test fonctionnel : audiogramme.

Une heure après le traumatisme, le composé (1) préserve 90 % de l'audition. Le temps auquel l'on peut donner le composé (1) après le traumatisme tout en préservant 50 % de l'audition est déterminé comme entre 6 à 7 heures. La fenêtre thérapeutique du composé (1) est de 24 heures après un traumatisme acoustique de120 db d'une durée de 30 minutes. C'est-à-dire que le composé (1) dans de ce modèle est actif dans les premières 24 heures après le trauma acoustique (cf. Figure 9 : Audiogrammes effectués 10 jours après un trauma sonore).

### Étude morphologique : histologie des cellules le long de la spirale de la cochlée un mois après le traumatisme acoustique.

La perfusion extra cochléaire de 300 µM de composé (1) démarrée six heures après le trauma acoustique protège encore une grande partie des cellules ciliées un mois après le trauma acoustique. En effet seulement 32 % des cellules ciliées internes et 18 % des cellules ciliées externes sont absentes dans la zone lésée par le trauma acoustique comparé au côté contra latéral exposé au bruit mais non traité par le composé (1) où 86 % des cellules ciliées internes et 62 % des cellules ciliées externes sont absentes dans la zone lésée par le trauma acoustique.

## Revendications

1. Utilisation d'un dérivé hétérocyclique répondant à la formule 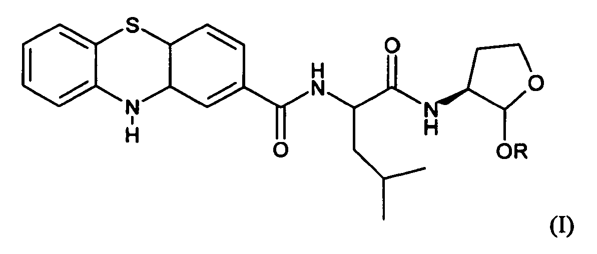 sous forme de diastéréoisomères ou toutes combinaisons de ces formes,
dans laquelle R représente l'atome d'hydrogène, un radical (C₁-C₆)alkyle, arylalkyle, ou -C(O)R' dans lequel R' représente un radical hétérocycloallcyle, (C₁-C₆)alkyle, aryle ou aralkyle ;
les radicaux alkyle, aryle ou hétérocycloalkyle étant éventuellement substitués par un ou plusieurs substituants identiques ou différents choisis parmi : (C₁-C₆)alkyle, hydroxy, (C₁-C₆)alkoxy, nitro, cyano, halogène ou NR₁R₂ ;
R₁ et R₂ représentent, indépendamment, un atome d'hydrogène ou un radical (C₁-C₆)alkyle, ou bien R₁ et R₂ forment ensemble avec l'atome d'azote auquel ils sont rattachés un hétérocycle éventuellement substitué,
pour la préparation d'un médicament destiné à prévenir et/ou traiter la perte d'audition.

2. Utilisation selon la revendication 1, **caractérisée en ce que** R représente -C(O)R'.

3. Utilisation selon la revendication 2, **caractérisée en ce que** R' représente un radical alkyle.

4. Utilisation selon la revendication 3, **caractérisée en ce** R représente -C(O)-CH₃.

5. Utilisation selon la revendication 1, **caractérisée en ce que** R représente l'hydrogène.

6. Utilisation selon l'une des revendications précédentes, **caractérisée en ce que** le composé (I) a pour formule

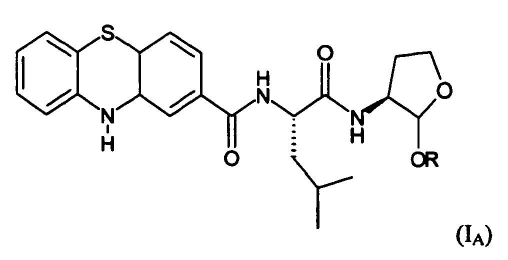

7. Utilisation selon la revendication 6, **caractérisée en ce que** le composé (I) a pour formule

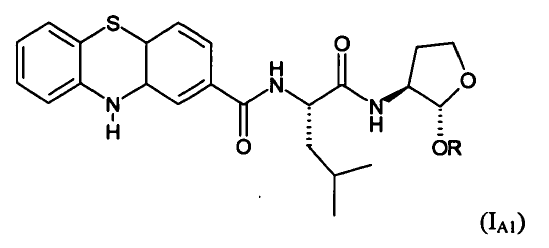

8. Utilisation selon la revendication 6, **caractérisée en ce que** le composé (I) a pour formule

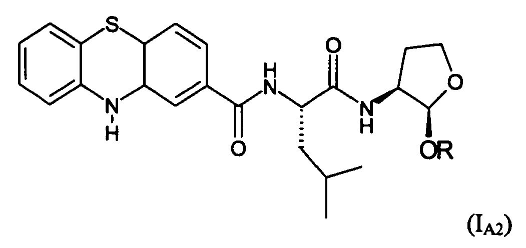

9. Utilisation selon l'une des revendications 1 à 5, **caractérisée en ce que** le composé (I) a pour formule

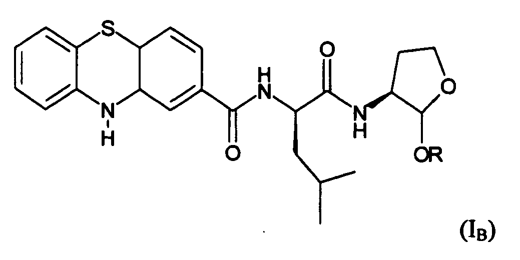

10. Utilisation selon la revendication 8, **caractérisée en ce que** le composé (I) a pour formule

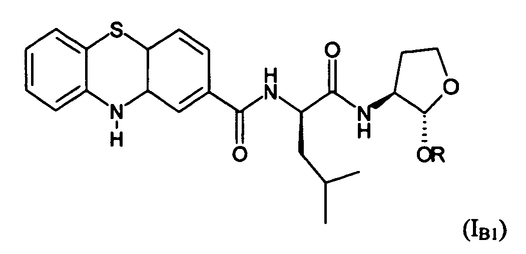

11. Utilisation selon la revendication 8, **caractérisée en ce que** le composé (I) a pour formule

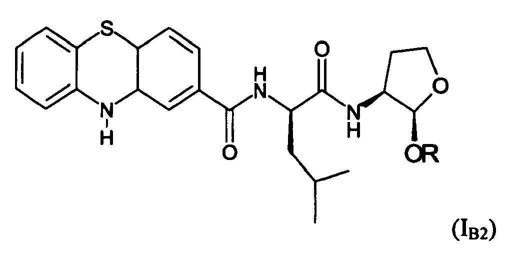

12. Utilisation selon l'une des revendications précédentes, **caractérisée en ce que** le dérivé (I) est à administrer en pré-traitement.

13. Utilisation selon l'une des revendications 1 à 11, **caractérisée en ce que** le dérivé (I) est à administrer en post-traitement.

14. Utilisation selon l'une des revendications précédentes, pour la préparation d'un médicament destiné à prévenir et/ou traiter la perte d'audition suite à l'administration d'un médicament.

15. Utilisation selon la revendication 14, suite à l'administration d'un autre médicament choisi parmi les antibiotiques, les anticancéreux, les agents anti-inflammatoires non stéroïdiens, les diurétiques, les anti-ulcéreux, les agents anticonvulsivants.

16. Utilisation selon la revendication 15, suite à l'administration d'un antibiotique.

17. Utilisation selon la revendication 16, **caractérisée en ce que** l'antibiotique est la gentamicine.

18. Utilisation selon l'une des revendications 1 à 13, pour la préparation d'un médicament destiné à prévenir et/ou traiter la perte d'audition suite à la presbyacousie.

19. Utilisation selon l'une des revendications 1 à 13, pour la préparation d'un médicament destiné à prévenir et/ou traiter la perte d'audition suite à un traumatisme acoustique.

20. Utilisation selon la revendication 19, **caractérisée en ce que** le composé (I) est à administrer dans les 7 heures et de préférence dans l'heure suivant le traumatisme acoustique.

21. Utilisation selon l'une des revendications précédentes, **caractérisée en ce que** le composé (I) est associé à au moins une autre substance à activité pharmaceutique choisie parmi : des anti-oxydants, des inhibiteurs de calpaïnes, des vasodilatateurs périphériques, des agonistes ou antagonistes du récepteur NMDA et des inhibiteurs peptidiques de c-Jun N-Terminal Kinase.

## Claims

1. Use of a heterocyclic derivative corresponding to the formula 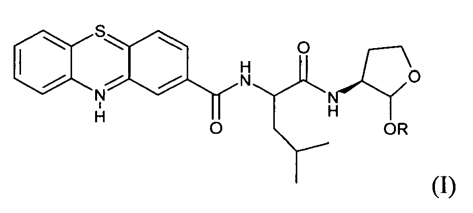 in the form of diastereoisomers or any combinations of these forms,
in which R represents the hydrogen atom, a (C₁-C₆)alkyl, arylalkyl or -C(O)R' radical
in which R' represents a heterocycloalkyl, (C₁-C₆)alkyl, aryl or aralkyl radical;
the alkyl, aryl or heterocycloalkyl radicals being optionally substituted by one or more identical or different substituants chosen from: (C₁-C₆)alkyl, hydroxy, (C₁-C₆)alkoxy, nitro, cyano, halogen or -NR₁R₂;
R₁ and R₂ represent, independently, a hydrogen atom or an (C₁-C₆)alkyl radical, or R₁ and R₂ form together with the nitrogen atom to which they are attached an optionally substituted heterocycle,
for the preparation of a medicament intended to prevent and/or treat hearing loss.

2. Use according to claim 1, **characterized in that** R represents -C(O)R'.

3. Use according to claim 2, **characterized in that** R' represents an alkyl radical.

4. Use according to claim 3, **characterized in that** R represents -C(O)-CH₃.

5. Use according to claim 1, **characterized in that** R represents hydrogen.

6. Use according to one of the preceding claims, **characterized in that** the compound (I) has the formula

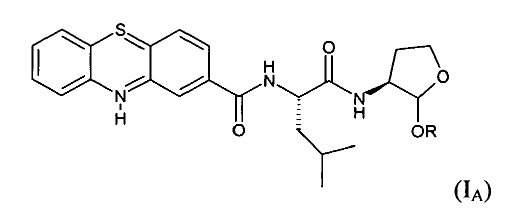

7. Use according to claim 6, **characterized in that** the compound (I) has the formula

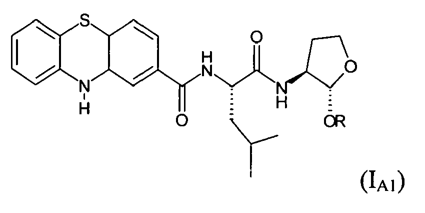

8. Use according to claim 6, **characterized in that** the compound (I) has the formula

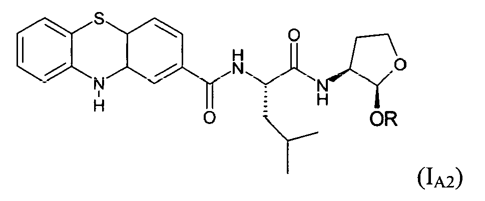

9. Use according to one of claims 1 to 5, **characterized in that** the compound (I) has the formula

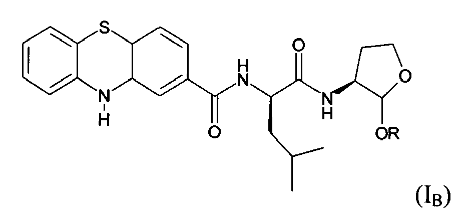

10. Use according to claim 8, **characterized in that** the compound (I) has the formula

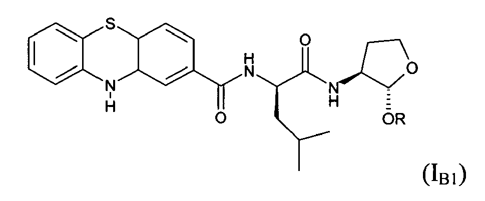

11. Use according to claim 8, **characterized in that** the compound (I) has the formula

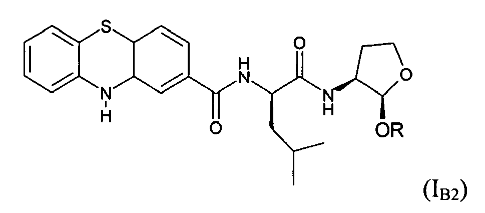

12. Use according to one of the preceding claims, **characterized in that** the derivative (I) is to be administered in a pre-treatment.

13. Use according to one of claims 1 to 11, **characterized in that** the derivative (I) is to be administered in a post-treatment.

14. Use according to one of the preceding claims, for the preparation of a medicament intended to prevent and/or treat hearing loss following the administration of a medicament.

15. Use according to claim 14, following the administration of another medicament chosen from antibiotics, anti-cancer drugs, non-steroidal anti-inflammatory agents, diuretics, antiulceratives, anticonvulsant agents.

16. Use according to claim 15, following the administration of an antibiotic.

17. Use according to claim 16, **characterized in that** the antibiotic is gentamicin.

18. Use according to one of claims 1 to 13, for the preparation of a medicament intended to prevent and/or treat hearing loss following presbycusis.

19. Use according to one of claims 1 to 13, for the preparation of a medicament intended to prevent and/or treat hearing loss following an acoustic traumatism.

20. Use according to claim 19, **characterized in that** the compound (I) is to be administered in the 7 hours and preferably in the hour following the acoustic traumatism.

21. Use according to one of the preceding claims, **characterized in that** the compound (I) is combined with at least one other substance with pharmaceutical activity chosen from: antioxidants, calpain inhibitors, peripheral vasodilitators, agonists or antagonists of the NMDA receptor and peptide inhibitors of c-Jun N-terminal kinase.

## Patentansprüche

1. Verwendung eines heterocyclischen Derivats, das der Formel 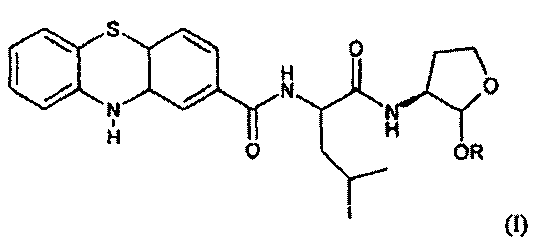 entspricht, in Form von Diastereoisomeren und allen Kombinationen dieser Formen,
in der R ein Wasserstoffatom, einen Rest (C₁-C₆)-Alkyl, Arylalkyl oder -C(O)R' darstellt, worin R' einen Rest Heterocycloalkyl, (C₁-C₆)-Alkyl, Aryl oder Aralkyl darstellt;
wobei die Alkyl-, Aryl- oder Heterocycloalkylreste gegebenenfalls mit einem oder mehreren gleichen oder verschiedenen Substituenten substituiert sind, die ausgewählt sind aus: (C₁-C₆)-Alkyl, Hydroxy, (C₁-C₆)-Alkoxy, Nitro, Cyano, Halogen oder-NR₁R₂;
R₁ und R₂ unabhängig voneinander ein Wasserstoffatom oder einen (C₁-C₆)-Alkylrest darstellen oder R₁ und R₂ zusammen mit dem Stickstoffatom, an das sie gebunden sind, einen gegebenenfalls substituierten Heterocyclus bilden,
für die Herstellung eines Medikaments, das für die Vorbeugung und/oder Behandlung des Hörverlusts bestimmt ist.

2. Verwendung nach Anspruch 1, **dadurch gekennzeichnet, dass** R -C(O)R' darstellt.

3. Verwendung nach Anspruch 2, **dadurch gekennzeichnet, dass** R' einen Alkylrest darstellt.

4. Verwendung nach Anspruch 3, **dadurch gekennzeichnet, dass** R -C(O)-CH₃ darstellt.

5. Verwendung nach Anspruch 1, **dadurch gekennzeichnet, dass** R Wasserstoff darstellt.

6. Verwendung nach einem der vorhergehenden Ansprüche, **dadurch gekennzeichnet, dass** die Verbindung (I) die Formel hat:

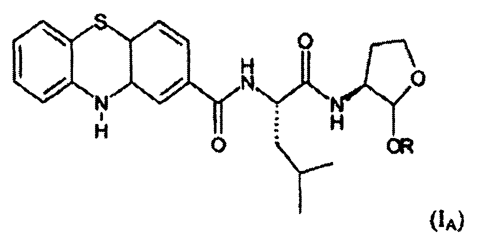

7. Verwendung nach Anspruch 6, **dadurch gekennzeichnet, dass** die Verbindung (I) die Formel hat:

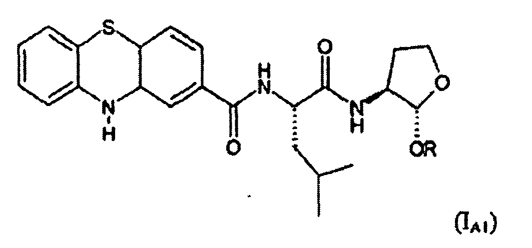

8. Verwendung nach Anspruch 6, **dadurch gekennzeichnet, dass** die Verbindung (I) die Formel hat:

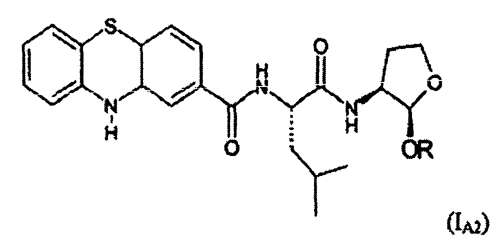

9. Verwendung nach einem der Ansprüche 1 bis 5, **dadurch gekennzeichnet, dass** die Verbindung (I) die Formel hat:

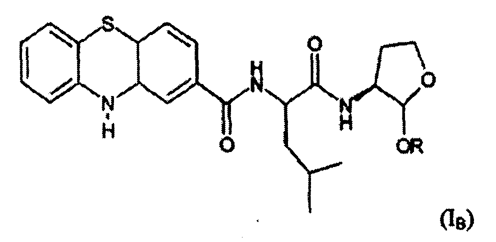

10. Verwendung nach Anspruch 8, **dadurch gekennzeichnet, dass** die Verbindung (I) die Formel hat:

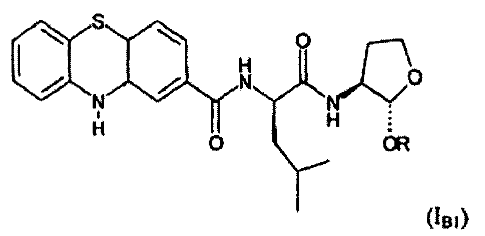

11. Verwendung nach Anspruch 8, **dadurch gekennzeichnet, dass** die Verbindung (I) die Formel hat:

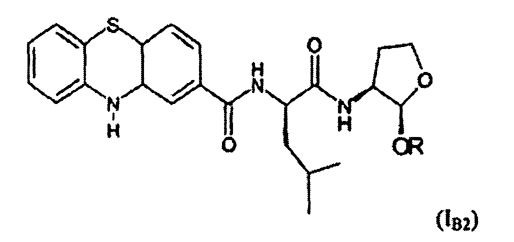

12. Verwendung nach einem der vorhergehenden Ansprüche, **dadurch gekennzeichnet, dass** das Derivat (I) in der Vorbehandlung zu verabreichen ist.

13. Verwendung nach einem der Ansprüche 1 bis 11, **dadurch gekennzeichnet, dass** das Derivat (I) in der Nachbehandlung zu verabreichen ist.

14. Verwendung nach einem der vorhergehenden Ansprüche für die Herstellung eines Medikaments, das für die Vorbeugung und/oder Behandlung des Hörverlusts infolge der Verabreichung eines Medikaments bestimmt ist.

15. Verwendung nach Anspruch 14 nach der Verabreichung eines anderen Medikaments, das aus den Antibiotika, den Anti-Krebs-Mitteln, den nichtsteroiden Antiphlogistika, den Diuretika, den Anti-Ulkus-Mitteln, den antikonvulsivisch wirkenden Mitteln ausgewählt ist.

16. Verwendung nach Anspruch 15 nach der Verabreichung eines Antibiotikums.

17. Verwendung nach Anspruch 16, **dadurch gekennzeichnet, dass** das Antibiotikum Gentamicin ist.

18. Verwendung nach einem der Ansprüche 1 bis 13 für die Herstellung eines Medikaments, das zur Vorbeugung und/oder Behandlung des Hörverlusts infolge von Presbyakusis bestimmt ist.

19. Verwendung nach einem der Ansprüche 1 bis 13 für die Herstellung eines Medikaments, das für die Vorbeugung und/oder Behandlung des Hörverlusts infolge eines akustischen Traumas bestimmt ist.

20. Verwendung nach Anspruch 19, **dadurch gekennzeichnet, dass** die Verbindung (I) innerhalb von 7 Stunden und vorzugsweise innerhalb einer Stunde nach dem akustischen Trauma zu verabreichen ist.

21. Verwendung nach einem der vorhergehenden Ansprüche, **dadurch gekennzeichnet, dass** die Verbindung (I) mit mindestens einer anderen Substanz mit pharmazeutischer Wirkung kombiniert ist, die ausgewählt ist aus: Antioxidantien, Calpainhemmer, periphere Vasodilatatoren, Agonisten oder Antagonisten des NMDA-Rezeptors und peptidische Hemmer der c-Jun N-Terminal Kinase.
